# EUROPÄISCHE PATENTSCHRIFT

(11) **EP 0 469 440 B1**
(45) Veröffentlichungstag und Bekanntmachung des Hinweises auf die Patenterteilung: **24.08.1994**
(21) Anmeldenummer: 91112368.5
(22) Anmeldetag: 24.07.1991
(51) Int. Cl.: A61K 9/20, A61K 9/48, A61K 31/675

(54) **Feste orale Applikationsformen, die als Wirkstoff Ifosfamid enthalten**
Solid oral administration forms containing ifosfamide as active agent
Formes solides d'administration orale contenant l'ifosfamide comme agent actif

(30) Priorität: 03.08.1990 DE 4024683
(43) Veröffentlichungstag der Anmeldung: 05.02.1992
(73) Patentinhaber: ASTA Medica Aktiengesellschaft, D-01277 Dresden (DE)
(72) Erfinder: Sauerbier, Dieter, W-4806 Werther (DE); Engel, Jòrgen, Dr., W-8755 Alzenau (DE); Milsmann, Eckhard, Dr., W-4800 Bielefeld (DE); Molge, Klaus, Dr., W-4800 Bielefeld (DE); Isaac, Otto, Dr., W-6450 Hanau (DE)

(56) Entgegenhaltungen:
- WO-A-89/07453
- US-A- 4 618 692

## Beschreibung

Ifosfamid ist die INN-Bezeichnung für 3-(2-Chlorethyl) -2-(chlorethylamino)-tetrahydro-2H-1,3, 2-oxazaphosphorin-2-oxid. Ifosfamid ist ein bedeutender zytostatisch wirksamer Arzneiwirkstoff vom Typ der Oxazaphosphorine.
Ifosfamid ist ein weißes, kristallines Pulver mit einem Schmelzpunkt von 48°C bis 51°C und stark hygroskopischen Eigenschaften. Bereits unterhalb des Schmelzpunktes beginnt Ifosfamid zu sintern; es muß deshalb bei möglichst niedrigen Temperaturen gelagert werden. Außerdem ist ein Kontakt mit Luftfeuchtigkeit möglichst zu vermeiden. Ifosfamid löst sich zu etwa 10 Gewichtsprozent in Wasser, ist aber in wäßriger Lösung nur begrenzt haltbar.
Bisher ist Ifosfamid nur in Zubereitungen zur parenteralen Anwendung zugelassen. Ifosfamid wird in Form eines Sterilkristallisates angeboten, das in Dosierungen von 200 mg bis 2000 mg in Injektionsflaschen abgefüllt ist. Vor der Applikation muß das Sterilkristallisat in Wasser für Injektionszwecke gelöst werden, so daß eine 4-%ige Konzentration nicht überschritten wird. Diese Lösung ist zur intravenösen Injektion geeignet. Zur intravenösen Kurzinfusion wird die Ifosfamid-Lösung in 500 ml Ringer-Lösung oder ähnlichen Infusionsflüssigkeiten aufgelöst. Die Infusionsdauer beträgt ca. 30 Minuten, eventuell 1 bis 2 Stunden. Bei der 24-Stunden-Infusion wird die Ifosfamid-Lösung beispielsweise in insgesamt 3 Liter 5 % Dextrose-Kochsalzlösung aufgelöst.

Ifosfamid verursacht bei der Herstellung und Verarbeitung mannigfaltige Probleme. Bei der Herstellung des steril kristallisierten Ifosfamids resultiert ein Produkt von wechselnder physikalischer Beschaffenheit. Durch die unterschiedliche Rieselfähigkeit wird insbesondere die Dosierungsgenauigkeit bei der Abfüllung in hohem Maße beeinträchtigt.
Die Verarbeitung des Ifosfamids wird weiterhin erschwert durch seine Hygroskopizität und den niedrigen Schmelzpunkt. Bei längerer Lagerung sintert das Sterilkristallisat und die Lösungsgeschwindigkeit vermindert sich. Mit beginnender Sinterung des Ifosfamids nehmen auch die Klarlöslichkeit und der pH-Wert der Lösung bei gleichzeitiger Gelbfärbung ab; eine therapeutische Verwendung ist dann im allgemeinen nicht mehr möglich.

Neben den Schwierigkeiten bei der Herstellung des Sterilkristallisates gibt es vor allen Dingen auch gravierende Nachteile bei der Anwendung. Die parenterale Anwendung kann nur von medizinischem Fachpersonal durchgeführt werden. Der Patient muß stationär in ein Krankenhaus aufgenommen werden oder sich zumindest täglich zur Behandlung einfinden. Dies bedeutet einen hohen Zeitaufwand für Personal und Patienten.
Die Herstellung der sterilen Injektionslösung aus Trockensubstanz macht aufgrund des Gefahrenpotentials der Substanz aufwendige Schutzmaßnahmen für das Personal erforderlich. Für einen Patienten ist die parenterale Therapie unangenehm, da er bei der Applikation einen schmerzhaften Einstich hinnehmen muß und für die Dauer der Infusion an ein Infusionsbesteck angeschlossen ist.

Aufgrund all dieser Nachteile besteht seit langem das Bedürfnis nach einer oralen Darreichungsform, welche die aufgezählten Nachteile nicht mehr aufweist. Mit einer oralen Applikation könnte eine ambulante Therapie durchgeführt werden. Eine orale Einnahme von Ifosfamid wäre für den Patienten angenehm und würde für das medizinische Personal kein Risiko mehr darstellen.

Alle Versuche, eine feste orale Form zu entwickeln scheiterten jedoch bisher an den beschriebenen physikalisch-chemischen Eigenschaften des Ifosfamids. Weichgelatinekapseln waren als Arzneiform nicht realisierbar. Offensichtlich reagierte hier der Wirkstoff mit der Kapselhülle, sie wurde gegerbt, die Kapsel löste sich nicht mehr im Magensaft auf. Ebenso schlugen viele Versuche zur Entwicklung einer Tablette bisher fehl. Die Substanz klebte an den Stempeln der Tablettenmaschine, die Tabletten waren zu weich, zum Teil spritzte der Wirkstoff beim Komprimieren verflüssigt aus der Matrize.

Überraschend wurde jetzt gefunden, daß Ifosfamid im Gemisch mit mikrokristalliner Cellulose in Hartgelatinekapseln abgefüllt werden kann. Überraschenderweise findet hier keine nachteilige Interaktion von Ifosfamid mit der Kapselhülle statt. Obwohl die Kapselhülle 12 % bis 15 % Wasser (Gewicht/Gewicht) enthält und Ifosfamid sowohl hygroskopisch als auch feuchtigkeitsempfindlich ist, erweist sich die gefüllte Hartgelatinekapsel über mehrere Jahre lagerfähig. Die Kapselhülle löst sich noch nach mehreren Jahre Lagerzeit innerhalb weniger Minuten im Magensaft auf.

Beispielsweise enthalten die erfindungsgemäßen Ifosfamid-Kapseln zwischen 100 mg bis 800 mg, vorzugsweise zwischen 200 mg bis 500 mg Ifosfamid.

Die Kapselmasse besteht im wesentlichen aus Ifosfamid und mikrokristalliner Cellulose; daneben enthält die Kapselmasse gegebenenfalls noch geringe Mengen an üblichen Fließregulier- und Gegenklebemittel. Diese Fließregulier- und Gegenklebemittel können einzeln oder im Gemisch zur Anwendung kommen. Die Gesamtmenge an solchen zusätzlichen Fließregulierungsmitteln und Gegenklebemittel bezogen auf 1 Gewichtsteil Ifosfamid beträgt zum Beispiel 0,001 bis 0,1 Gewichtsteile, vorzugsweise 0,01 bis 0,04 Gewichtsteile. Als solche Fließregulier- und Gegenklebemittel kommen beispielsweise solche in Frage, die zum Beispiel in folgenden Lehrbüchern aufgeführt sind:
W.A. Ritschel, DIE TABLETTE, Editio Cantor Verlag, Seite 125, 1. Auflage 1966 Sucker, Fuchs, Speiser, PHARMAZEUTISCHE TECHNOLOGIE, G. Thieme Verlag, Stuttgart, Seite 334 bis 336, 1. Auflage 1978
Münzel, Büchi, Schultz, GALENISCHES PRAKTIKUM, Wissenschaftliche Verlagsgesellschaft Stuttgart, Seite 731, 1. Auflage 1959
R. Voigt, LEHRBUCH DER PHARMAZEUTISCHEN TECHNOLOGIE, 4. Auflage, Verlag Chemie, Weinheim, Seite 195, 1. Auflage 1982
P.H. List, ARZNEIMITTELLEHRE, Wissenschaftliche Verlagsanstalt, Stuttgart, Seite 86, 1. Auflage 1976
Insbesondere kommen in Frage Magnesiumstearat, sowie andere Stearate, hochdisperses Siliciumdioxid, Stearinsäure, Talkum und Polyglykole (zum Beispiel mit Molgewichten von 4000 bis 6000).

Vorzugsweise wurden als Fließregulierungsmittel 0,002 bis 0,02 Gewichtsteile, insbesondere 0,005 bis 0,008 Gewichtsteile pro 1 Gewichtsteil Ifosfamid und als Gegenklebemittel 0,004 bis 0,08 Gewichtsteile, insbesondere 0,016 bis 0,032 Gewichtsteile pro 1 Gewichtsteil Ifosfamid verwendet.

Ferner können die Kapseln gegebenenfalls noch enthalten Füllmittel wie Stärke, Cellulose, Milchzucker, Fructose, Saccharose, Mannit, Sorbit, Calciumphosphat, Bindemittel wie Gelatine, Cellulose, Pektine, Alginate, Polyvinylpyrroliodon, Sprengmittel wie Alginate, Carboxymethylcellulosen, Polyvinylpyrrolidone, Ultraamylopektin.

Als Fließreguliermittel kommt insbesondere hochdisperses Siliciumdioxid (zum Beispiel Aerosil® wie Aerosil® V 200) sowie Magnesiumstearat in Frage.

Die Menge an mikrokristalliner Cellulose in der erfindungsgemäßen Kapsel beträgt im allgemeinen 0,2 bis 4 Gewichtsteile, vorzugsweise 0,25 bis 1 Gewichtsteil, insbesondere 0,3 bis 0,35 Gewichtsteile bezogen auf 1 Gewichtsteil Ifosfamid. Die verwendete mikrokristalline Cellulose soll hinsichtlich der Kristallinität einen Kristallinitätsindex* zwischen 0,5 bis 0,9 beispielsweise von 0,7 aufweisen. Der Polymerisationsgrad der mikrokristallinen Cellulose liegt vorzugsweise im Bereich von 200 bis 300. Weiterhin soll die erfindungsgemäß verwendete mikrokristalline Cellulose beispielsweise eine mittlere Korngröße von ca. 50 »m beziehungsweise unter 50 »m aufweisen. Beispielsweise liegt diese unter 40 »m, insbesondere bei 20 »m. Vorzugsweise wird als mikrokristalline Cellulose Avicel® verwendet, z.B. Avicel® mit einem Korngrößenspektrum von kleiner als 38 »m (Avicel® PH 105) (das heißt mindestens 90 % der mikrokristallinen Cellulose haben eine mittlere Teilchengröße von kleiner als 38 »m, insbesondere 20 »m).
* Unter Kristallinitätsindex versteht man den Quotienten aus kristallinem Anteil und der Summe von krsitallinem und amorphem Anteil. Für kristalline Cellulose mit einer Korngröße von ca. 50 »m beträgt der Index-Wert zum Beispiel 0,71.

Weiterhin gelang es jetzt überraschenderweise auch, Tabletten mit dem Wirkstoff Ifosfamid herzustellen, wobei die Kombination von Tricalciumphosphat und Polyethylenglykol von besonderer Bedeutung ist. Durch diese Maßnahmen ist nun erstmalig das Verpressen auf einer herkömmlichen Tablettenpresse möglich.

Die Substanz Ifosfamid kann aufgrund ihrer physikalischen Eigenschaften nicht in herkömmlicher Art direkt auf Tablettenmaschinen zu Tabletten verpreßt werden. Alle Versuche, den Wirkstoff unter Verwendung der bekannten Hilfsstoffe wie zum Beispiel mikrokristalline Cellulose, Lactose, Stärke, Talkum, hochdisperses Siliciumdioxid, Calciumhydrogenphosphat zu verpressen scheiterten. Auch Versuche mit Hilfe von Granulationen in konventioneller Art oder in der Wirbelschicht führten nicht zu Tablettenmassen, die sich einwandfrei verarbeiten ließen. In allen Fällen wurde ein starkes Kleben der Masse beim Preßvorgang am Stempelwerkzeug beziehungsweise an der Matrize festgestellt.

Die erfindungsgemäßen Tabletten enthalten, bezogen auf einen Gewichtsteil Ifosfamid,
0,1 - 1,0 Gewichtsteile Tricalclumphosphat und
0,04 - 0,4 Gewichtsteile Polyethylenglykol (zum Beispiel Molgewicht 4000 bis 6000)
sowie zusätzlich, bezogen auf das Tablettengewicht
5 - 60 Gewichts% eines Füll- und Fließregulierungsmittels
1 - 10 Gewichts% eines Sprengmittels
0,1 - 10 Gewichts% eines Gegenklebemittels und
0,1 - 80 Gewichts% eines Bindemittels.

Pro 1 Gewichtsteil Ifosfamid werden erfindungsgemäß zum Beispiel eingesetzt:
0,1 - 1,0 Gewichtsteile, vorzugsweise 0,2 - 0,5, insbesondere 0,25 - 0,30 Gewichtsteile Tricalciumphosphat. Bezogen auf das Tablettengemisch ist die Menge an Tricalciumphosphat zum Beispiel 3,5 bis 35 Gewichts%, vorzugsweise 7 bis 17,8 Gewichts%, insbesondere 9 bis 11 Gewichts%.

Die Menge an Polyethylenglykol ist beispielsweise 0,04 - 0,4 Gewichtsteile, vorzugsweise 0,1 - 0,2, insbesondere 0,13 - 0,15 Gewichtsteile pro 1 Gewichtsteil Ifosfamid. Es kommt insbesondere Polyethylenglykol mit Molgewichten von 4000 bis 6000, vorzugsweise Polyethylenglvkol 6000 in Frage. Bezogen auf das Tablettengemisch ist die Menge an Polyethylenglykol zum Beispiel 1 bis 14.0 Gewichts%, vorzugsweise 3,5 bis 7,5 Gewichts%, insbesondere 4,5 bis 7 oder auch 4,5 bis 6 Gewichts%. Das Gewichtsverhältnis von Tricalciumphosphat zu Polyethylenglykol ist beispielsweise 1 : 0,5.

Zusätzlich sind in der erfindungsgemäßen Tablette noch enthalten:
Füll- und Fließregulierungsmittel in einer Menge von 5 bis 60 Gewichts%, bezogen auf das Tablettengewicht. Als Füllmittel kommen zum Beispiel in Frage Stärken, Cellulosen, Lactose, Saccharose, Fructose, Sorbit, Mannit, Calciumphosphat, Calciumcarbonat, Calciumsulfat, Magnesiumcarbonat oder -oxid. Es werden 5 - 60 Gewichts%, bezogen auf das Tablettengewicht, eingesetzt.
Als Fließregulierungsmittel kommen zum Beispiel in Frage mikrokristalline Cellulose, Lactose, Polyglykole, Stärken, Cellulosen, Talcum, Talcum siliconisatum, Calciumarachinat oder -stearat, Cetylalkohol, Stearylalkohol, Myristylalkohol, Stearinsäure, Laurinsäure. Falls das Fließregulierungsmittel nicht zugleich auch als Füllmittel dient, werden hiervon 0,5 - 10 Gewichts%, bezogen auf das Tablettengewicht, eingesetzt.
Sprengmittel: Zum Beispiel Alginate, Stärken (Malsstärke), Pektine, Carboxymethylcellulosen, Polyvinylpolypyrrolidon, Ultraamylopectin, Betonite. Es werden 1 - 10 Gewichts%, bezogen auf das Tablettengewicht, eingesetzt.
Gegenklebemittel: Zum Beispiel Glykole, Talcum, Talcum siliconisatum, Talcum stearinicum, Calciumstearat, Aluminiumstearat, Stearinsäure. Es werden 0,1 - 10 Gewichts%, bezogen auf das Tablettengewicht, eingesetzt.
Bindemittel: Zum Beispiel Gelatine, Celluloseether, Amylose, Pektine, Cellulose, Dextrose, Polyglykole, Traganth. Es werden 0,1 - 80 Gewichts%, bezogen auf das Tablettengewicht, eingesetzt.

Insbesondere enthält die erfindungsgemäße Tablette außer Ifosfamid, Tricalciumphosphat und Polyethylenglykol die folgenden Stoffe:
Mikrokristalline Cellulose 0,2 - 1,2 Gewichtsteile, vorzugsweise 0,4 - 1,0, insbesondere 0,70 - 0,90 Gewichtsteile, bezogen auf einen Gewichtsteil Ifosfamid oder bezogen auf das Tablettengewicht 7 bis 43, vorzugsweise 15 bis 35 Gewichts%;
Lactose 0,15 - 1,0 Gewichtsteile, vorzugsweise 0,24 - 0,68, insbesondere 0,30 - 0,40 Gewichtsteile, bezogen auf einen Gewichtsteil Ifosfamid oder bezogen auf das Tablettengewicht 5,0 bis 36, vorzugsweise 8,5 bis 25 Gewichts%;
Maisstärke 0,02 - 0,24 Gewichtsteile, vorzugsweise 0,05 - 0,20, insbesondere 0,1 - 0,15 Gewichtsteile, bezogen auf einen Gewichtsteil Ifosfamid oder bezogen auf das Tablettengewicht 0,7 bis 8,5, vorzugsweise 2,0 bis 6,5 Gewichts%;
Talkum 0,02 - 0,30 Gewichtstelle, vorzugsweise 0,06 - 0,20, insbesondere 0,07 - 0,09 Gewichtsteile, bezogen auf einen Gewichtsteil Ifosfamid oder bezogen auf das Tablettengewicht 0,70 bis 10, vorzugsweise 2 bis 6,5 Gewichts%;
Magnesiumstearat 0,004 - 0,2 Gewichtsteile, vorzugsweise 0,02 - 0,12, insbesondere 0,035 - 0,05 Gewichtsteile, bezogen auf einen Gewichtsteil Ifosfamid oder bezogen auf das Tablettengewicht 0,1 bis 7,2, vorzugsweise 0,7 bis 4,5 Gewichts%.

Sowohl Tabletten als auch Kapseln können in bekannter Weise mit einem Überzug versehen werden. Es kann sich hierbei um wasserlösliche, quellbare, wasserunlösliche oder magensaftresistente Überzüge handeln, die aus wäßriger Dispersion oder Lösung oder auch aus Lösung oder Dispersion in organischen Lösungsmitteln wie zum Beispiel Ethanol, Isopropanol, Aceton, Ether, Dichlormethan, Methanol auf die Tabletten oder Kapseln aufgebracht werden.

Die Herstellung der Kapseln und Tabletten erfolgt beispielsweise zwischen 15°C und 26°C, vorzugsweise zwischen 18°C und 22°C. Die relative Feuchte in den Produktionsräumen soll 40 % nicht überschreiten.

Die Herstellung der erfindungsgemäßen festen oral einzunehmenden Ifosfamid-Zubereitungen erfolgt dadurch, daß man zwischen 15 °C und 30 °C entweder einen Gewichtsteil Ifosfamid mit 0,1 - 4 Gewichtsteilen, vorzugsweise 0,2 - 4, insbesondere 0,25 - 1 Gewichtsteilen mikrokristalliner Cellulose sowie gegebenenfalls geringen Mengen eines üblichen Fließregulierungs- und Gegenklebemittels homogen vermischt und in Kapseln abfüllt oder daß man einen Gewichtsteil Ifosfamid mit
0,01 - 1,0 Gewichtsteilen Tricalciumphosphat,
0,04 - 0,4 Gewichtsteilen Polyethylenglykol sowie
0,15 - 2, vorzugsweise 0,5 - 1,5, insbesondere 1 - 1,3 Gewichtsteilen eines Füll- und Fließregulierungsmittels,
0,03 - 0,5, vorzugsweise 0,05 - 0,4, insbesondere 0,08 - 0,2 Gewichtsteilen eines Sprengmittels,
0,003 - 0,5, vorzugsweise 0,01 - 0,4, insbesondere 0,05 - 0,2 Gewichtsteilen eines Gegenklebemittels und
0,003 - 3, vorzugsweise 0,01 - 2, insbesondere 0,1 -1 Gewichtsteilen eines Bindemittels
homogen vermischt und anschließend zu Tabletten verpreßt und gegebenenfalls die erhaltenen Kapseln oder Tabletten mit einem üblichen Überzug versieht.

### Beispiel 1:

### Ifosfamid-Kapselmasse

Die Herstellung der Kapselmasse erfolgt erfindungsgemäß zum Beispiel nach folgender Methode:
Für 12.000 Kapseln a 250 mg werden zum Beispiel 3,0 kg Ifosfamid, 1,002 kg mikrokristalline Cellulose und 0,018 kg hochdisperses Siliciumdioxid durch ein 0,8 mm-Sieb gegeben und anschließend in einem geeigneten Mischer 4 Minuten gemischt. Anschließend werden dieser Mischung 0,06 kg Magnesiumstearat hinzugefügt (gesiebt durch 0,8 mm Sieb) und nochmals 1 Minute gemischt. Die fertige Kapselmasse wird auf einer mit Formatteilen der Größe 1 ausgerüsteten Kapselmaschine in Hartgelatinekapseln der Größe 1 gefüllt, so daß jede Kapsel ca. 340 mg der Kapselmasse enthält.

Für 20.000 Kapseln a 500 mg werden zum Beispiel 10,0 kg Ifosfamid, 3,34 kg mikrokristalline Cellulose und 0,06 kg hochdisperses Siliciumdioxid durch ein 0,8 mm Sieb gegeben und anschließend in einem geeigneten Mischer 4 Minuten gemischt. Anschließend werden dieser Mischung 0,2 kg Magnesiumstearat hinzugefügt (gesiebt durch 0,8 mm Sieb) und nochmals 1 Minute gemischt. Die fertige Kapselmasse wird auf einer mit Formatteilen der Größe 00 ausgerüsteten Kapselmaschine in Hartgelatinekapseln der Größe 00 gefüllt, so daß jede Kapsel ca. 680 mg der Kapselmasse enthält. Die mikrokristalline Cellulose wird zum Beispiel in Form von Avicel PH 105 eingesetzt. Avicel PH 105 besitzt ein besonderes Korngrößensprektrum und stellt einen Füllstoff mit gutem Binde- und Fließvermögen dar.

Zur Herstellung von magensaftresistenten Kapseln wird beispielsweise auf 2500 Kapseln der Größe 1 mit einem Wirkstoffgehalt von 250 mg Ifosfamid eine Menge von 3000 g einer Überzugssuspension in organischem Lösungsmittel (Isopropanol) aufgetragen. Die 3000 g Suspension enthalten:
1440 g Anionisches Polymerisat aus Methacrylsäure und Methacrylsäureestern mit einem mittleren
Molekulargewicht von beispielsweise 150 000, denen ein üblicher Weichmacher zugesetzt ist, 18 g 1,2-Propandiol, 36 g Magnesiumstearat sowie 1506 g Isopropanol.
Als Copolymerisat aus Methacrylsäure und Methylmethacrylat kommt beispielsweise Eudragit L®in Frage, insbesondere in Form einer 12,5 %igen Lösung in Isopropanol (Eudragit L®/12,5 %ig). Solche Copolymerisate sind durch Salzbildung mit Alkalien in neutralem bis schwach alkalischem Milieu löslich.

### Beispiel 2:

### Ifosfamid-Tablette

Die Rezeptur für eine Tablette mit einem Wirkstoffgehalt von 250 mg setzt sich beispielsweise wie folgt zusammen:
Eine Tablette zu 700 g enthält:

| | |
|---|---|
| Ifosfamid | 250 mg |
| Tricalciumphosphat, fein | 70 mg |
| Mikrokristalline Cellulose | 200 mg |
| Lactose | 85 mg |
| Polyglykol 6000 | 35 mg |
| Maisstärke | 30 mg |
| Talkum | 20 mg |
| Magnesiumstearat | 10 mg |

Zur Herstellung der Tablettenmasse für 1500 Tabletten werden 375 g Ifosfamid, 105 g Tricalciumphosphat fein, 300 g mikrokristalline Cellulose, 127,5 g Lactose, 52,5 g Polyglykol 6000, 45 g Maisstärke, 30 g Talkum durch ein Sieb der Maschenweite 0,8 mm passiert und in einem geeigneten Mischer 15 Minuten lang gemischt. Anschließend werden die ebenfalls gesiebten 15 g Magnesiumstearat zugegeben und nochmals 2 Minuten gemischt. Die Tablettenmasse wird anschließend auf einer geeigneten Tablettenpresse zu Tabletten verpreßt.

Zur Herstellung von magensaftresistent überzogenen Tabletten werden auf 1050 g Tabletten beispielsweise 500 g der im folgenden beschriebenen wäßrigen Dispersion aufgetragen:
100 g der wäßrigen Dispersion enthalten:

| | |
|---|---|
| Polyglykol 6000 | 1,600 g |
| Titandioxid | 1,100 g |
| Eisenoxid gelb | 0,156 g |
| Talkum | 4,000 g |
| Dimethylpolysiloxan | 0,100 g |
| Eudragit L® 30 D* | 55,000 g |
| Wasser | 38,044 g |
| | 100,000 g |

| | |
|---|---|
| * Eudragit L® 30 D ist die wässrige Dispersion eines Copolymerisats mit anionischem Charakter auf Basis von Methacrylsäure und Ethylacrylat. Das Verhältnis der freien Carboxylgruppen zu den Estergruppen beträgt etwa 1 : 1. Das mittlere Molekulargewicht liegt bei 250 000. | |

Das Aufsprühen der Filmlösung erfolgt zum Beispiel in hierfür üblichen Apparaturen, in dem die Lösungs- bzw. Dispersionsmittel durch Trocknung kontinuierlich entfernt werden.

## Patentansprüche

1. Feste orale Ifosfamid-Zubereitungen, dadurch gekennzeichnet, daß es sich um Ifosfamid-Kapseln handelt, wobei die Kapselmasse im wesentlichen aus dem Wirkstoff Ifosfamid und mikrokristalliner Cellulose besteht, gegebenenfalls neben geringeren Mengen eines üblichen Fließregulierungs- und Gegenklebemittels, oder daß es sich um Tabletten handelt, die bezogen auf einen Gewichtsteil Ifosfamid,
0,1 - 1,0 Gewichtsteile Tricalciumphosphat und
0,04 - 0,4 Gewichtsteile Polyethylenglykol
enthalten sowie zusätzlich, bezogen auf das Tablettengewicht
5 - 60 Gewichts% eines Füll- und Fließregulierungsmittels
1 - 10 Gewichts% eines Sprengmittels
0,1 - 10 Gewichts% eines Gegenklebemittels und
0,1 - 80 Gewichts% eines Bindemittels aufweisen.

2. Verfahren zur Herstellung von festen oral einzunehmenden Ifosfamid-Zubereitungen, dadurch gekennzeichnet, daß man zwischen 15 °C und 30 °C entweder einen Gewichtsteil Ifosfamid mit 0,1 - 4 Gewichtsteilen mikrokristalliner Cellulose sowie gegebenenfalls geringen Mengen eines üblichen Fließregulierungs- und Gegenklebemittels homogen vermischt und in Kapseln abfüllt oder daß man einen Gewichtsteil Ifosfamid mit
0,1 - 1,0 Gewichtsteilen Tricalciumphosphat,
0,04 - 0,4 Gewichtsteilen Polyethylenglykol sowie
0,15 - 2 Gewichtsteilen eines Füll- und Fließregulierungsmittels,
0,03 - 0,5 Gewichtsteilen eines Sprengmittels,
0,003 - 0,5 Gewichtsteilen eines Gegenklebemittels und
0,003 - 3 Gewichtsteilen eines Bindemittels homogen vermischt und anschließend zu Tabletten verpreßt und gegebenenfalls die erhaltenen Kapseln oder Tabletten mit einem üblichen Überzug versieht.

## Claims

1. A solid oral ifosfamide formulation, characterized in that it takes the form of ifosfamide capsules, the weight of the capsule consisting essentially of the active ingredient, ifosfamide, and microcrystalline cellulose, optionally with additional smaller amounts of a conventional flow regulator and antiadhesion agent, or in that it takes form of tablets containing, based on one part by weight of ifosfamide, 0.1 - 1.0 part by weight of tricalcium phosphate and 0.04 - 0.4 part by weight of polyethylene glycol, and additionally containing, based on the weight of the tablet,
5 - 60% by weight of a filler and flow regulator,
1 - 10% by weight of a disintegrating agent,
0.1 - 10% by weight of an antiadhesion agent and
0.1 - 80% by weight of a binder.

2. A process for the preparation of solid ifosfamide formulations for oral administration, characterized in that, between 15°C and 30°C, either one part by weight of ifosfamide is homogeneously mixed with 0.1 - 4 parts by weight of microcrystalline cellulose and optionally small amounts of a conventional flow regulator and antiadhesion agent and filled into capsules, or one part by weight of ifosfamide is homogeneously mixed with
0.1 - 1.0 part by weight of tricalcium phosphate,
0.04 - 0.4 part by weight of polyethylene glycol,
0.15 - 2 parts by weight of filler and flow regulator,
0.03 - 0.5 part by weight of an antiadhesion agent and 0.003 - 3 parts by weight of a binder
and then compressed to tablets and, optionally, the capsules or tablets obtained are provided with a conventional coating.

## Revendications

1. Préparations solides d'ifosfamide pour administration orale, caractérisées en ce qu'il s'agit de capsules d'ifosfamide, la masse des capsules se composant essentiellement de la substance active ifosfamide et de cellulose microcristalline, en dehors éventuellement de petites quantités d'un agent usuel de réglage de la fluidité et antiagglutinant, ou bien en ce qu'il s'agit de comprimés qui contiennent, par rapport à 1 partie en poids d'ifosfamide,
0,1 - 1,0 partie en poids de phosphate tricalcique et
0,04 - 0,4 partie en poids de polyéthylèneglycol
et qui comportent en plus, par rapport au poids du comprimé,
5 - 60% en poids d'une matière de charge et de réglage de la fluidité,
1 - 10% en poids d'un désintégrant,
0,1 - 10% en poids d'un antiagglutinant et
0,1 - 80% en poids d'un liant.

2. Procédé de confection de préparations solides d'ifosfamide pour administration orale, caractérisé en ce qu'entre 15°C et 30°C, ou bien on mélange de façon homogène 1 partie en poids d'ifosfamide avec 0,1-4 parties en poids de cellulose microcristalline et éventuellement avec de petites quantités d'un agent usuel de réglage de la fluidité et antiagglutinant et on remplit des capsules avec le mélange, ou bien on mélange de façon homogène 1 partie en poids d'ifosfamide avec
0.1 - 1,0 partie en poids de phosphate tricalcique,
0,04 - 0,4 partie en poids de polyéthylèneglycol, ainsi qu'avec
0,15 - 2 parties en poids d'une substance de charge et de réglage de la fluidité,
0,03 - 0,5 partie en poids d'un désintégrant,
0,003 - 0,5 partie en poids d'un antiagglutinant et
0,003 - 3 parties en poids d'un liant,
puis on presse le mélange en comprimés,
et on garnit éventuellement les capsules ou les comprimés obtenus d'un enrobage usuel.
